# EUROPEAN PATENT SPECIFICATION

(11) **EP 4 265 666 B1**
(45) Date of publication and mention of the grant of the patent: **27.11.2024**
(21) Application number: 22169072.0
(22) Date of filing: 20.04.2022
(51) Int. Cl.: C08G 65/26

(54) **DIBORANE-BASED, METAL-FREE CATALYST SYSTEMS FOR THE PREPARATION OF ISOTACTIC-ENRICHED ALIPHATIC POLYETHERS, RELATED PROCESS, POLYETHER OBTAINED BY THE PROCESS AND USE OF THE CATALYST SYSTEMS**
DIBORANBASIERTE METALLFREIE KATALYSATORSYSTEME FÜR DIE HERSTELLUNG VON ISOTAKTISCH ANGEREICHERTEN ALIPHATISCHEN POLYETHERN, ZUGEHÖRIGES VERFAHREN, AUS DEM VERFAHREN ERHALTENER POLYETHER UND VERWENDUNG DES KATALYSATORSYSTEMS
SYSTÈMES CATALYSEURS SANS MÉTAL À BASE DE DIBORANE POUR LA PRÉPARATION DE POLYÉTHERS ALIPHATIQUES ENRICHIS EN ISOTACTIQUE, PROCÉDÉ ASSOCIÉ, POLYÉTHER OBTENU PAR LE PROCÉDÉ ET L'UTILISATION DES SYSTÈMES CATALYSEURS

(43) Date of publication of application: 25.10.2023
(73) Proprietor: Universität Stuttgart, 70174 Stuttgart (DE)
(72) Inventor: NAUMANN, Stefan, 73733 Esslingen am Neckar (DE); SIRIN-SARIASLAN, Ayla, 97922 Lauda-Königshofen (DE); BUCHMEISER, Michael, 73630 Remshalden (DE)
(74) Representative: Meissner Bolte Partnerschaft mbB

(56) References cited:
- SIRIN-SARIASLAN AYLA ET AL: "Chiral Diboranes as Catalysts for the Stereoselective Organopolymerization of Epoxides", CHEMICAL SCIENCE, 31 August 2022 (2022-08-31), United Kingdom, XP055957540, ISSN: 2041-6520, Retrieved from the Internet <URL:https://pubs.rsc.org/en/content/articlepdf/2022/sc/d2sc03977j> [retrieved on 20220905], DOI: 10.1039/D2SC03977J
- PERETTI KATHRYN L. ET AL: "A Highly Active, Isospecific Cobalt Catalyst for Propylene Oxide Polymerization", JOURNAL OF THE AMERICAN CHEMICAL SOCIETY, vol. 127, no. 33, 26 July 2005 (2005-07-26), pages 11566 - 11567, XP055957622, ISSN: 0002-7863, Retrieved from the Internet <URL:https://pubs.acs.org/doi/pdf/10.1021/ja053451y> [retrieved on 20220905], DOI: 10.1021/ja053451y
- CHEN YE ET AL: "High Efficiency Organic Lewis Pair Catalyst for Ring-Opening Polymerization of Epoxides with Chemoselectivity", MACROMOLECULES, vol. 51, no. 20, 12 October 2018 (2018-10-12), US, pages 8286 - 8297, XP055927147, ISSN: 0024-9297, Retrieved from the Internet <URL:https://pubs.acs.org/doi/pdf/10.1021/acs.macromol.8b01852> [retrieved on 20220905], DOI: 10.1021/acs.macromol.8b01852

## Description

The invention relates to a metal-free ("organocatalytic") catalyst setup for the synthesis of isotactic and isotactic enriched (*it*) polyethers (proportion of isotactic diad placement (*m*) > 50%). Therein, the catalyst setup comprises an organobase (BA) in combination with a Lewis acid (LA) in the form of a chiral diborane species. The invention is further concerned with a process for the production of said *it*-polyethers via the stereocontrolled ring-opening polymerization of epoxides using the above described catalyst system and various protic initiators (IN), in particular also enabling the introduction of *it-*polyether blocks in more complex polymer architectures such as block copolymers containing polyester and *it*-polyether blocks.

### Background of the invention

Polyethers are widely employed polymers, which are utilized for example in pharmaceutical applications, as lubricants, rheology controllers, surfactants or as macromonomers for poly(urethane) synthesis. Larger scale preparation and products of commercial significance to date exclusively resort to *atactic* (*at*) variants of polyether. However, it has been shown that control of tacticity is a key tuning means to impact polyether properties and applicability. Highly isotactic polyether derived from substituted epoxides is usually semi-crystalline with sometimes surprisingly high melting points, in contrast to the fully amorphous, atactic congeners (J. Am. Chem. Soc. 2010, 132, 16520-16525). Also, *it-*poly(propylene oxide) (PPO) was found to combine high tensile strength and photodegradability, potentially recommending this material for use as non-persistent fibre material in marine applications (J. Am. Chem. Soc. 2020, 142, 6800-6806). Further, it was shown that the rheology of aqueous solutions can be modulated by adjusting the tacticity of polyether-based additives (Macromol. Chem. Phys. 2020, 1900437). To realize such potentials, simple and powerful catalytic systems, able to generate it-enriched polyether from racemic epoxide monomer, are needed. In particular, the incorporation of well-defined *it*-polyether moieties in complex polymer architectures, such as poly(ester-block-*it* PPO), has not been described before. Moreover, metal-free polymerization setups (asymmetric organocatalysis) for this purpose is absent from research literature so far.

Past research has focused on the development of metal complexes to generate it-polyethers, often focussing on PO as monomer.

Early patenting activity describes the Pruitt-Baggett catalyst (Pruitt, M. E.; Baggett, J. M. U.S. Patent 2,706,181, 1955**;** Pruitt, M. E.; Baggett, J. M.; Bloomfield, R. J.; Templeton, J. H. U.S. Patent 2,706,182, 1955**;** Pruitt, M. E.; Baggett, J. M. U.S. Patent 2,706,189, 1955**),** which consists of FeCl₃ reacted with PO and diethyl ether to result in a brown residue capable of forming a mixture of semicrystalline optically active material and amorphous inactive material.

In the 1960s, Vandenberg described that mixtures of aluminiumorganyls (AlR₃), water and acetylacetonate (Vandenberg, E. J. J. Polym. Sci. 1960, 47, 486; Vandenberg, E. J. U.S. Patent 3,135,705, 1964**;** Vandenberg, E. J. U.S. Patent 3,219,591, 1965**)** yield a catalytically active compound to prepare polyether with high molar mass and moderate isotacticity. The molecular structure of the catalyst is still under debate.

Also aluminium-porphyrin complexes were found to exhibit a moderate selectivity, as described by Inoue (m = 69%, Makromol. Chem. 1978, 179, 1377).

The application of a Grignard-type reagent, Mg(nBu)₂, was demonstrated to result in poly(PO), whereby molar masses of up to 65,000 g/mol and considerable isoselectivity were achieved *(*Macromolecules 2017, 50, 1245-1250). Molar mass distribution was moderately controlled (*Ð* _{M} = 1.6 - 2.3).

A range of bimetallic Co(III) and Cr (III) catalysts was disclosed by Coates and co-workers (J. Am. Chem. Soc. 2005, 127, 11566-11567; J. Am. Chem. Soc. 2008, 130, 17658-17659; J. Am. Chem. Soc. 2010, 132, 16520-16525; Angew. Chem. Int. Ed. 2018, 57, 5731 -5734), giving access to *it*-PPO and other polyethers with very high selectivity; block copolymers with non-polyether blocks were not disclosed.

As outlined, the prior art relies exclusively on metal-based catalysts for the stereoselective polymerization of epoxides. Metal-free approaches, including boranes, have so far only addressed the generation of atactic polyethers such as at-PPO:
The scientific publications Macromolecules 2018, 51, 8286 and ChemSusChem 2018, 24, 4209 describe a combination of triethyl borane and phosphazene bases for PO polymerization in the presence of alcohol initiators. Molecular weights of up to *M*ₙ = 200,000 g/mol are described. No stereoselectivity is found.

In Acc. Chem. Res. 2021, 54, 4434, various applications of achiral di-, tri- and tetraboranes for polymerization purposes, including epoxide polymerization, are described. No stereoselective behaviour is disclosed.

The objective of the present invention was thus to identify an optimized and simple catalyst system for the synthesis of *it*-enriched polyethers via ROP of racemic epoxides, wherein the catalyst system should be characterized by a ready accessibility, metal-free character and tolerance towards non-ether building blocks such as polyesters, allowing for the construction of *it*-polyether containing polymer architectures.

Surprisingly, it has been found that the problem can be solved by a catalyst system which comprises a range of applicable organobases (B) in combination with simple, chiral diborane Lewis acids (LA). This allows for tailoring different degrees of isotacticity (isotactic diad placement *m* = 50-100%), achieving high molar masses and narrow molar mass distributions (*M*ₙ up to > 100.000 g/mol, *Ð* _{M} < 1.4) and enabling the realization of (multi-)block copolymers containing polyester and *it*-enriched polyether blocks.

### Description of the invention:

In a first embodiment, the present application is therefore directed at a catalyst system for the synthesis of *it*-polyethers from *rac*-epoxide monomers in the presence of an initiator IN, wherein the catalyst system comprises an organobase BA and a diborane Lewis acid LA.

In a preferred embodiment, the catalyst system of the invention comprises
a mixture of organobase BA and diborane Lewis acid LA having the general formula (I), 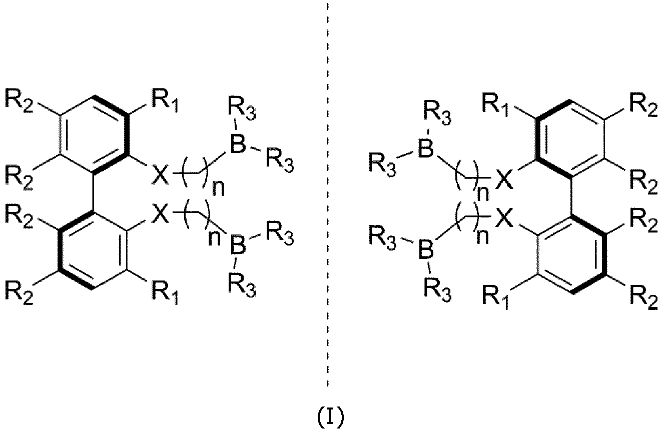
wherein R₁ mutually independently represents H, C₁-C₁₀ alkyl, C₂-C₁₀ alkenyl, C₃-C₁₂ cycloalkyl, C₆-C₁₀₀ polyoxyalkylene, C₅-C₁₀ aryl or C₅-C₁₀ heteroaryl, wherein the C₅-C₁₀ aryl or C₅-C₁₀ heteroaryl may be further substituted;
R₂ and R₃ mutually independently represent H, C₁-C₁₀ alkyl, C₂-C₁₀ alkenyl, C₃-C₁₂ cycloalkyl, C₆-C₁₀₀ polyoxyalkylene, C₅-C₁₀ aryl or C₅-C₁₀ heteroaryl, wherein the C₅-C₁₀ aryl or C₅-C₁₀ heteroaryl may be further substituted;
wherein if the C₅-C₁₀ aryl or C₅-C₁₀ heteroaryl mentioned above is further substituted, the further substituent is preferably a phenyl- or an alkyl- and more preferably a C₁₋₆-alkyl substituent, wherein preferably the substituted C₅-C₁₀ aryls or C₅-C₁₀ heteroaryls preferably have 1 to 3 substituents;
wherein X is -O-, -S-, -CH₂-, -NR₅- or -PR₅, and R₅ has the meaning given above for R₁;
wherein n determines the number of methylene (-CH₂-) repeat units, wherein preferably n is an integer from 1 to 10.

The diborane Lewis acid in the catalyst can either be enantiomerically pure, a racemic mixture or a mixture of R and S enantiomers in any ratio. In the context of this invention, a racemic mixture is a mixture of R and S enantiomers in a ratio of about 50:50. An enantiomerically pure diborane Lewis acid for the purposes of this invention is considered as a mixture of S and R enantiomers where one enantiomer is present in an excess over the other enantiomer in a ratio of at least 9:1. In a preferred embodiment, the diborane Lewis acid in the catalyst is enantiomerically pure or a racemic mixture.

In the diborane Lewis acid of the formula (I) it is preferred that R₁ mutually independently represents H, methyl, ethyl, n-propyl, isopropyl, tert-butyl, neopentyl, isoamyl, cyclohexyl, phenyl, 2,6-dimethylphenyl, 2,6-diisopropylphenyl, 3,5-diphenylphenyl or mesityl moieties. More preferably all R¹ are selected from H, t-butyl, phenyl and 3,5-diphenylphenyl. In a particularly preferred embodiment all R¹ are same residue.

In addition or in alternative thereto, it is preferred that R₂ in the formula (I) mutually independently represents H, methyl, ethyl, n-propyl, isopropyl, tert-butyl or the two R₂ together with the phenyl, to which they are attached form a naphthyl group, wherein more preferably all R₂ in the formula (I) form naphthyl groups. More preferably, R² mutually independently represents H, methyl or the two R₂ together with the phenyl, to which they are attached form a naphthyl group. In a particularly preferred embodiment all R² are same residue.

In addition or in alternative thereto, it is preferred that R₃ mutually independently represents H, methyl, ethyl, n-propyl, isopropyl, tert-butyl or, together with the B-atom to which they are attached form a borabicyclo[3.3.1]nonane group. More preferably, R³ together with the B-atom to which they are attached form a borabicyclo[3.3.1]nonane group.

X in the diborane Lewis acid of the formula (I) preferably mutually independently represents O or CH₂.

n preferably mutually independently represents 1 or 3 methylene (-CH₂-) units, and more preferably 1 or 2 methylene units.

In a yet even more preferred embodiment of the invention, the diborane Lewis acid (LA) is one or more compounds having the formulae (II-1), (II-2), (II-3), (II-4), (II-5), (II-6), (II-7) and (II-8) shown below, whereby these structures preferably are either enantiomerically pure or racemic compounds.

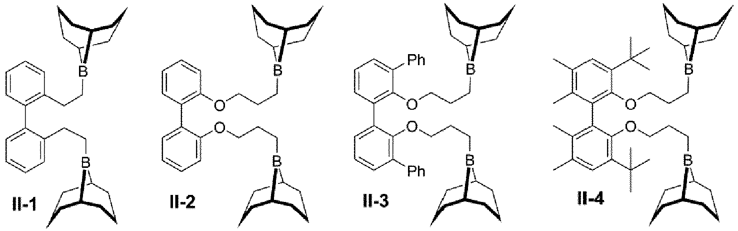

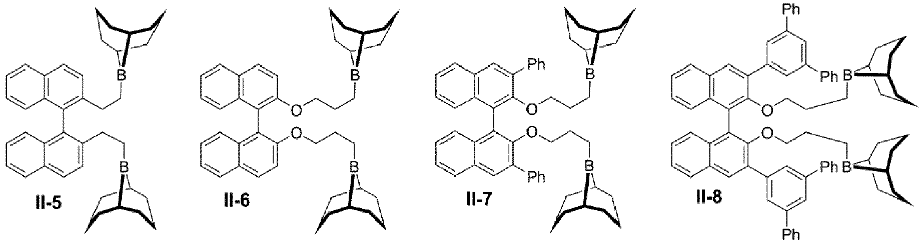

The term "organobase BA" as used herein refers to a metal-free, neutral Brönsted base, the latter defined according to IUPAC as "a molecular entity capable of accepting a hydron (proton) from an acid (i.e. a 'hydron acceptor') or the corresponding chemical species".

The organobase in the catalyst system of the invention regularly comprises functionalities such as tertiary amines, amidines, guanidines, alkoxides, amides, hydrides, *N*-heterocyclic carbenes, *N*-heterocyclic olefins or phosphazenes.

In a preferred embodiment, the organobase BA in the catalyst system is one or more compounds selected from (III-1), (III-2), (III-3), (III-4), (III-5), (III-6), (III-7), (III-8), (III-9), (III-10), (III-11), (III-12), (III-13), (III-14), (III-15), (III-16), (III-17), (III-18), (III-19), (III-20), (III-21) and (III-22) shown below.

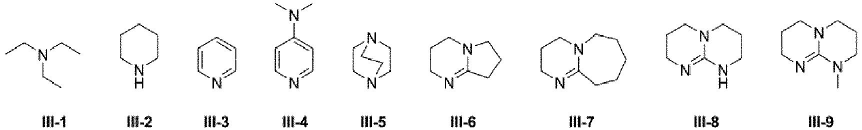

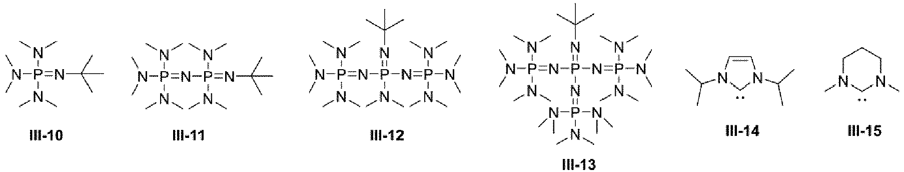

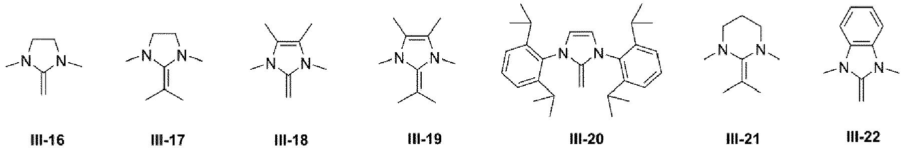

The term initiator IN refers to a protic compound on which the (*it*-enriched) polyether moiety will be grown. The initiator preferably has the general formula (IV),

R-YH (IV)

wherein Y is -O-, -S-, -NH- or COO and R is selected from polymeric residues such as in particular polyethers, polyesters, or polyamides, and/or wherein R is selected from C₁-C₁₀ alkyl, C₂-C₁₀ alkenyl, C₃-C₁₂ cycloalkyl, C₆-C₁₀₀ polyoxyalkylene, C₅-C₁₀ aryl, benzyl or C₅-C₁₀ heteroaryl, wherein the C₅-C₁₀ aryl or C₅-C₁₀ heteroaryl may be further substituted.

In a preferred embodiment, IN comprises benzyl alcohol, butan(di)ol, Methoxypolyethylenglycol (mPEG), HO-PEG-OH (preferably with an Mₙ in the range of from 100 to 35.000 g/mol) or polycaprolactone-OH, and particularly preferably consists of either of these compounds. The Mₙ and the molecular weight in general in this application is determined by GPC as noted in the experimental section below.

In one embodiment, the initiator is not part of the catalyst system of the invention, in which case the catalyst system is limited to the components which are catalytically active in the provision of polyethers. In another embodiment, the initiator is part of the catalyst system of the invention, so that the system is specifically adapted to provide polyethers; in the course of the production of the polyethers, the initiator is then incorporated therein.

As concerns the molar ratio of LA to BA, the catalyst system of the invention is not particularly limited. Preferably, however, the molar ratio of the organobase to the diborane Lewis acid to is in the range from 1 : 0.5 to 1 : 50, more preferably in the range from 1 : 0.5 to 1 : 10, and even more preferably in the range from 1 : 1 to 1 : 4. As concerns the ratio of IN to either BA or LA, there is no particular limitation except that molar ratio of IN to BA should be > 1. In a preferred embodiment, the molar ratio of IN to either BA or AL is 50:1 to 1:1, in a yet more preferred embodiment 25:1 to 1:1 and most preferred the ratio is in the range of from 5:1 to 1:1.

As noted above the catalyst system is different form catalyst systems for the production of *it*-polyethers in that it does not require the use of metals. Thus, in a preferred embodiment, the catalyst system of the invention is substantially metal free and does not comprises metal additives, which are deliberately added (in this case, metals may only be present in quantities of inevitable impurities).

As indicated above, the catalyst systems of the invention are particularly effective for the production of *it*-polyethers by ring-opening of (substituted) epoxide monomers. Therefore, in another embodiment of the invention is directed at a process for the production of polyethers, wherein an epoxide compound (or "epoxide monomer") is reacted in the presence of a catalyst system as described above, i.e. a catalyst system, which is based on a combination of an organobase BA and a diborane Lewis acid LA in the presence of initiator IN.

In a preferred embodiment of the process according to the invention the epoxide compound comprises one or more compounds selected from the group consisting of 4-tert-butylphenyl glycidyl ether, phenyl glycidyl ether, 1-naphthyl glycidyl ether, 2-naphthyl glycidyl ether, 4-chlorophenyl glycidyl ether, 2,4,6-trichlorophenyl glycidyl ether, 2,4,6-tribromophenyl glycidyl ether, pentafluorophenyl glycidyl ether, cyclohexyl glycidyl ether, benzyl glycidyl ether, glycidyl benzoate, glycidyl acetate, glycidyl cyclohexylcarboxylate, methyl glycidyl ether, ethyl glycidyl ether, butyl glycidyl ether, hexyl glycidyl ether, 2-ethylhexyl glycidyl ether, octyl glycidyl ether, C10 - C18 alkyl glycidyl ether, allyl glycidyl ether, ethylene oxide, propylene oxide, styrene oxide, 1,2-butene oxide, 2,3-butene oxide, 1,2-hexene oxide, oxides of C10 - C18 alpha-olefins, cyclohexene oxide, vinylcyclohexene monoxide, limonene monoxide, and/or butadiene monoepoxide.

In a preferred embodiment of the invention, the epoxide compound is selected from the group consisting of ethylene oxide, propylene oxide, butylene oxide, hexylene oxide, allyl glycidyl ether, 2-ethylhexyl glycidyl ether. Most preferred as epoxide compound are propylene oxide and/or butylene oxide.

Concerning the molar ratio of epoxide compound to the diborane Lewis acid LA in the catalyst system, the invention is not particularly limited, except that the molar amount of the diborane Lewis acid LA should be significantly (i.e. at least 5 times) lower than the molar amount of the epoxide compound. Preferably, the molar ratio of epoxide compound to LA is at least about 50 : 1, more preferably at least about 250 : 1 and most preferable at least about 500 : 1. Similarly, the epoxide/BA molar ratio is at least 100 : 1, more preferably 500 : 1 and most preferable 1000: 1. The epoxide/IN molar ratio is not particularly limited and is preferably in the range of 10 : 1 to 1000 : 1.

The process according to the invention is performed either in bulk monomer or in non-protic solvents.

Suitable non-protic solvents are for example linear or branched alkanes or mixtures of alkanes, toluene, xylene and the isomeric xylene mixtures, mesitylene, mono- or polysubstituted halogenated aromatic solvents or halogenated alkane solvents, for example chlorobenzene, dichlorobenzene, dichloromethane (DCM), dichloroethane, tetrachloroethane, linear or cyclic ether such as tetrahydrofurane (THF) or methyl-tert-butylether (MTBE) or higher ether of the repeating unit R'-(CH₂CH₂O)ₙ-R with n>1 where R and R' display a aliphatic moiety like methyl, ethyl, n-propyl, 2-propyl, n-butyl, 2-butyl, 1,1-dimethylethyl, linear or cyclic ester, or polar aprotic solvents such as 1,4-dioxane, dimethylsulfoxide (DMSO), sulfolane or mixtures of the above mentioned solvents and/or with other solvents. Preferred non-protic solvents are pentane, toluene, THF and DCM.

The reaction of the epoxide compound may be carried out in a continuous process, in a batch process or in a semi-batch process.

In one embodiment of the process according to the invention, the process is carried out continuously. That means in this continuous process the epoxide compound, the catalyst combination and, if required, the solvent are continuously added whereas a part of the reaction mixture is continuously removed from the reactor. A residence-time reactor may be added after the continuous reactor in order to complete the reaction.

In an alternative embodiment, the process according to the invention is carried out as a batch process. In this batch process the epoxide monomer, the catalyst combination, and, if necessary, the solvent are charged in a reactor and the reaction runs until full conversion is obtained.

In a yet alternative embodiment, the process according to the invention is carried out as a semi-batch process. In the semi-batch process, the epoxide compound is preferably mixed with a catalyst and optionally a solvent in a reactor and further epoxide monomer is continuously added to the reaction as pure material or in solution.

The reaction temperature is not subject to any relevant restrictions, except that the temperature should not be too high to negate it-selectivity of the catalyst setup. As a suitable reaction temperature, a range of -78°C to 50°C can be mentioned. Preferably, the reaction temperature is in the range from -35°C to 25°C.

A suitable reaction time for the process of the invention is for example 0.05 to 240 hours, preferably 0.25 to 96 hours, and more preferred 0.5 to 24 hours. The reaction time is the time wherein the epoxide, the catalyst system and the solvent are in direct contact at the reaction temperature.

In an embodiment, the process according to the invention further comprises a step of isolating the *it*-polyether or it-polyether containing product from the reaction mixture, and heating and pressing it into a desired shape.

If the polyether comprises units derived from more than one precursor monomer, the distribution of the monomers may be block-like, gradient, or fully random.

The process according to the invention is suited for the synthesis of polyethers with interesting properties for use, for example, as pharmaceutics, drug-delivery agents, medical gels, rheology modifiers or low-temperature duty rubbers.

In a yet further aspect, the present invention is directed to the use of a catalyst system as described above for the production of *it*-polyethers. The use generally involved contacting the catalyst system, an initiator IN and an epoxy monomer at a temperature sufficiently high for polymerisation and sufficiently low to provide in isotactic (it) polyether.

Polyethers obtained by the method according to the invention are particularly suited as polymer building blocks in polyurethane (PU) chemistry. For example, OH-terminated polyethers may be reacted with isocyanates to form foams or other polyurethanes. The degree of tacticity may thereby be used to impact the properties of the resulting polymers, for example targeting "soft-segmented" PU (low %*m*) or "hard-segmented" PU (high %m). The *it*-polyethers are further especially suited for use as lubricants, surfactants and solvents with modified properties. High molar mass, highly isotactic PPO may also be used as photodegradable, non-toxic and non-persistent fibre material.

Further possible uses of the polyethers according to the invention include for example:
- Lubricants, suitable for low- and high-temperature application.
- Surfactants, including for washing applications, anti-foaming agents in technical and food applications
- Rheology modifiers
- Plasticizers, softening additives for polymer blends
- Anti-freeze agents
- Structure-directing agents in self-assembly processes, for example for the preparation of mesoporous materials
- Damping materials, rubber and rubber-like materials
- Medical gels, especially injectable gels
- Solid or semi-solid electrolytes in electrochemical devices, such as lithium-ion or lithium-sulphur batteries
- Thermally conductive formulations for thermal management applications like heatsinks.
- Food contact applications (coatings, emulsifiers)
- Lubricating coating for surfaces in aqueous and non-aqueous environments
- Excipient in pharmaceutical applications, also as drug-delivery agent
- Stationary phase in (gas) chromatography
- Preservation applications, including wood
- Solvent for chemical reactions, or solvent for ink in printers
- Polyols, for example for polyurethane production
- Preparation of micelle- and /or polymersome-containing solutions
- Cosmetic applications, including as basis of creams or as dispersants in toothpaste
- Membranes
- Binder for ceramics production

The present invention will be further described with reference to the following examples without wishing to be limited by them.

### Examples

### Epoxide compound (M)

| | |
|---|---|
| **PO** | Propylene oxide, obtained from TCI (> 99%). |
| **BO** | 1-Butylene oxide, obtained from TCI (> 99%). |
| **AGE** | Allyl glycidylether, obtained from ABCR (99%) |

Monomers were dried over CaH₂, distilled under nitrogen and degassed (freeze-pump-thaw) or used as received prior to storing inside a glove box (N₂, -35°C).

### Diborane Lewis acid LA

The compounds **II-2, II-3, II-4, II-6-, II-7, II-8** were synthesized starting from commercial 2,2'-binaphthol derivatives (BINOLs), followed by Williamson etherification (with allyl bromide) and subsequent hydroboration using 9-BBN. Syntheses were conducted on a multigram scale. The identity of the compounds was confirmed by a combination ¹H, ¹¹B and ¹³C-NMR as well as fine mass analysis and X-ray single crystal structure analysis.

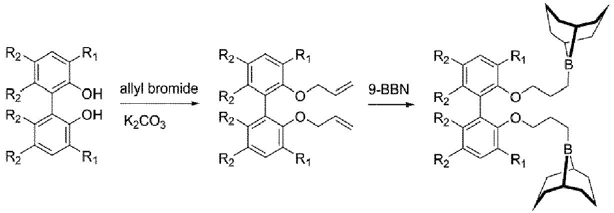

The compounds **11-1, II-5** were synthesized from commercial 2,2'-dibromo binaphthyl derivatives, followed by reaction with n-butyl lithium/dimethylformamide to introduce aldehyde motifs and subsequent Wittig reaction and hydroboration. Syntheses were conducted on a multigram scale. The identity of the compounds was confirmed by a combination ¹H, ¹¹B and ¹³C-NMR as well as fine mass analysis and X-ray single crystal structure analysis.

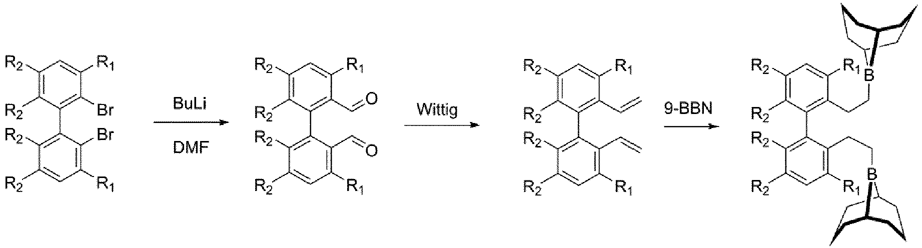

The diborane Lewis acids LA were stored under nitrogen inside the glove box.

### Organobases BA

| | |
|---|---|
| **III-7** | 1,8-Diazabicyclo[5.4.0]undec-7-ene (DBU), Aldrich, used as received |
| **III-11** | 1-*tert*-Butyl-2,2,4,4,4-pentakis-(dimethylamino)-2Λ5,4Λ5-catenadiphosphazene (tBu-P₂), 2M in THF, Aldrich, used as received |
| **III-19** | Prepared according to Eur. J. Inorg. Chem. 2013, 2013, 2301-2314 |

All BA components were stored under protective conditions (nitrogen).

### Initiators IN

| | |
|---|---|
| **BnOH** | Benzyl alcohol, Aldrich, used as received |
| **PCL-OH** | Polycaprolactone (hydroxyl capped), Aldrich, *M*ₙ = 10.000 g/mol, *Ð*_{M} = 1.45 |

### Solvents

Toluene and THF were dried using a solvent purification system (SPS) by MBraun and stored inside a glove box under protective gas and over molecular sieve.

Molecular sieves (3 Å, Honeywell) were activated in a vacuum oven at 130°C for 24 h prior to use.

The reactions were accomplished according to the below reaction scheme:

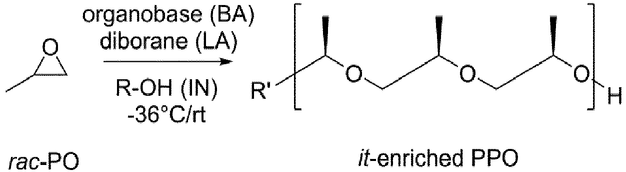

All polymerisations were assembled under protective atmosphere.

### Characterisation of polyethers

¹H nuclear magnetic resonance (NMR) analysis was employed to investigate conversion, while ¹³C-NMR was used to elucidate tacticity (isotactic diad placement, *m*). NMR (400 MHz, 300 K): ¹H/¹³C NMR spectra were recorded on a Bruker Avance III 400 spectrometer, with the chemical shifts being reported relative to reference peaks of the applied deuterated solvents (CDCl₃: δ = 7.26/77.16 ppm for proton and carbon spectra, respectively).

Gel permeation chromatography (GPC) was used to determine the molecular weight (number average molecular weight, *M*ₙ) and polydispersity (*M*_{w}/*M*ₙ). GPC (CHCl₃, 40 °C): A chromatographic assembly comprising a PSS SDV 5 µm 8*50mm guard column, three PSS SDV 100 000 Å 5 µm 8*50mm columns and an Agilent 1200 Series G1362A detector (RI) was employed. The concentration of the prepared samples amounted to 2.5 mg/mL, and a flow-rate of 1 mL/min was applied during the analyses. Polystyrene standards were used for calibration.

### Reactor

Small scale reactions were conducted in 4-20 mL glass vials equipped with a magnetic stirring bar and sealed with a cap; the vial was placed on a magnetic stirrer inside the glove box freezer if reactions were to be conducted at -36°C. All reactions were conducted under nitrogen, with a moisture-free atmosphere.

**Table 1: Summary of examples for the synthesis of it-polyethers via ring-opening polymerization of epoxides using the inventive catalyst system. IN = BnOH (except sample 6), no solvent (except samples 6 to 10 and 12).**

| # | Epox y (M) | BA | LA | BA/LA/IN/M (molar) | *t* [h] | *T* [°C ] | Conv [%] | *m* [%] | *M*ₙ (PDI) [g/mol] |
|---|---|---|---|---|---|---|---|---|---|
| 1 | PO | **III-7** | **II-1** | 1:2:5:2000 | 24 | 25 | 32 | 66 | 3.000 (1.4) |
| 2 | PO | **III-7** | **II-2** | 1 :4:5:2000 | 24 | -36 | 70 | 57 | 19.000 (1.26) |
| 3 | PO | **III-11** | **II-4** | 1 :4:5:2000 | 72 | -36 | 70 | 59 | 16.000 (1.09) |
| 4 | PO | **III-19** | **II-5** | 1:4:2.5:5000 | 20 | -36 | 23 | 72 | 38.000 (1.32) |
| 5 | PO | **III-11** | **(*R*)-II-6** | 1:2:5:2000 | 72 | -36 | 75 | 68 | 24.000 (1.16) |
| 6^{a,b)} | PO | **III-19** | **(*R*)-II-6** | 1:2:2.5:1000 | 48 | 25 | 53 | 80 | 38.000 (1.24) |
| 7b) | PO | **III-19** | **(*R*)-II-6** | 1:2:2.5:1000 | 24 | -36 | 80 | 82 | 26.000 (1.14) |
| 8b) | PO | **III-19** | **(*R*)-II-6** | 1:2:2.5:10000 | 24 | -36 | 52 | 79 | 111.000 (1.18) |
| 9^{b)} | BO | **III-19** | **(*R*)-II-6** | 1:2:2.5:1000 | 10d | -36 | 13 | 84 | 4.000 (1.13) |
| 10^{b)} | AGE | **III-19** | **(*R*)-II-6** | 1:2:2.5:10000 | 2 w | -36 | 9 | 75 | 13.000 (1.33) |
| 11 | PO | **III-11** | **(*R*)-II-7** | 1 :4:5:2000 | 2 | 25 | 63 | 70 | 14.000 (1.3) |
| 12^{c)} | PO | **III-19** | **(*R*)-II-7** | 1 :2:2.5: 1000 | 8 d | 25 | 16 | 88 | 5.000 (1.14) |
| 13 | BO | **III-19** | **(*R*)-II-7** | 1:2:2.5:1000 | 24 | 25 | 14 | 75 | 3.000 (1.17) |
| 14 | AGE | **III-19** | **(*R*)-II-7** | 1:2:2.5:1000 | 24 | 25 | 11 | 64 | 4.000 (1.21) |

| | | | | | | | | | |
|---|---|---|---|---|---|---|---|---|---|
| a) IN = PCL; b) solvent = toluene; c) solvent = THF | | | | | | | | | |

### Example 1:

First the diborane **11-1** (0.017 mmol, 2 eq.) was dissolved in PO (0.987 g, 17.04 mmol, 2000 eq.). Subsequently, the initiator (BnOH, 0.0425 mmol, 5 eq.) and the corresponding organobase **(III-7,** 0.0085 mmol, 1 eq.) were added to result in a total molar ratio of BA/LA/IN/PO = 1:2:5:2000. The reaction was stirred for 24 h at T = 25°C. The reaction was quenched by evaporation and subjected to NMR and GPC analysis.

### Example 2:

First the diborane **II-2** (0.034 mmol, 4 eq.) was dissolved in PO (0.987 g, 17.04 mmol, 2000 eq.). Subsequently, the initiator (BnOH, 0.0425 mmol, 5 eq.) and the corresponding organobase **(III-7,** 0.0085 mmol, 1 eq.) were added to result in a total molar ratio of BA/LA/IN/PO = 1:4:5:2000. The reaction was stirred for 24 h at T = -36°C. The reaction was quenched by evaporation and subjected to NMR and GPC analysis.

### Example 3:

First the diborane **II-4** (0.034 mmol, 4 eq.) was dissolved in PO (0.987 g, 17.04 mmol, 2000 eq.). Subsequently, the initiator (BnOH, 0.0425 mmol, 5 eq.) and the corresponding organobase **(III-11,** 0.0085 mmol, 1 eq.) were added to result in a total molar ratio of BA/LA/IN/PO = 1:4:5:2000. The reaction was stirred for 72 h at T = -36°C. The reaction was quenched by evaporation and subjected to NMR and GPC analysis.

### Example 4:

First the diborane **II-5** (0.034 mmol, 4 eq.) was dissolved in PO (2.46 g, 42.6 mmol, 5000 eq.). Subsequently, the initiator (BnOH, 0.0213 mmol, 2.5 eq.) and the corresponding organobase **(III-19,** 0.0085 mmol, 1 eq.) were added to result in a total molar ratio of BA/LA/IN/PO = 1:4:2.5:2000. The reaction was stirred for 20 h at T = -36°C. The reaction was quenched by evaporation and subjected to NMR and GPC analysis.

### Example 5:

First the diborane **(*R*)-II-6** (0.017 mmol, 2 eq.) was dissolved in PO (0.987 g, 17.04 mmol, 2000 eq.). Subsequently, the initiator (BnOH, 0.0425 mmol, 5 eq.) and the corresponding organobase **(III-11,** 0.0085 mmol, 1 eq.) were added to result in a total molar ratio of BA/LA/IN/PO = 1:2:5:2000. The reaction was stirred for 72 h at T = -36°C. The reaction was quenched by evaporation and subjected to NMR and GPC analysis.

### Example 6:

First the diborane **(*R*)-II-6** (0.017 mmol, 2 eq.) was dissolved in PO (0.495 g, 8.52 mmol, 1000 eq.) and Toluene (4.27 mL). Subsequently, the initiator (PCL, 0.0213 mmol, 2.5 eq.) and the corresponding organobase **(III-19,** 0.0085 mmol, 1 eq.) were added to result in a total molar ratio of BA/LA/IN/PO = 1:2:2.5:1000 and an initial monomer concentration of 2mol/L. The reaction was stirred for 48 h at T = 25°C. The reaction was quenched by evaporation and subjected to NMR and GPC analysis.

### Example 7:

First the diborane **(*R*)-II-6** (0.017 mmol, 2 eq.) was dissolved in PO (0.495 g, 8.52 mmol, 1000 eq.) and Toluene (4.27 mL). Subsequently, the initiator (BnOH, 0.0213 mmol, 2.5 eq.) and the corresponding organobase **(III-19,** 0.0085 mmol, 1 eq.) were added to result in a total molar ratio of BA/LA/IN/PO = 1:2:2.5:1000 and an initial monomer concentration of 2mol/L. The reaction was stirred for 24 h at T = -36°C. The reaction was quenched by evaporation and subjected to NMR and GPC analysis.

### Example 8:

First the diborane **(*R*)-II-6** (0.017 mmol, 2 eq.) was dissolved in PO (4.95 g, 85.2 mmol, 10000 eq.) and Toluene (42.7 mL). Subsequently, the initiator (BnOH, 0.0213 mmol, 2.5 eq.) and the corresponding organobase **(III-19,** 0.0085 mmol, 1 eq.) were added to result in a total molar ratio of BA/LA/IN/PO = 1:2:2.5:10000 and an initial monomer concentration of 2mol/L. The reaction was stirred for 24 h at T = -36°C. The reaction was quenched by evaporation and subjected to NMR and GPC analysis.

### Example 9:

First the diborane **(*R*)-II-6** (0.017 mmol, 2 eq.) was dissolved in BO (0.740 g, 8.52 mmol, 1000 eq.) and Toluene (4.27 mL). Subsequently, the initiator (BnOH, 0.0213 mmol, 2.5 eq.) and the corresponding organobase **(III-19,** 0.0085 mmol, 1 eq.) were added to result in a total molar ratio of BA/LA/IN/PO = 1:2:2.5:1000 and an initial monomer concentration of 2mol/L. The reaction was stirred for 10 d at T = -36°C. The reaction was quenched by evaporation and subjected to NMR and GPC analysis.

### Example 10:

First the diborane **(*R*)-II-6** (0.017 mmol, 2 eq.) was dissolved in AGE (10.02 g, 85.2 mmol, 10000 eq.) and Toluene (42.7 mL). Subsequently, the initiator (BnOH, 0.0213 mmol, 2.5 eq.) and the corresponding organobase **(III-19,** 0.0085 mmol, 1 eq.) were added to result in a total molar ratio of BA/LA/IN/PO = 1:2:2.5:10000 and an initial monomer concentration of 2mol/L. The reaction was stirred for 14 d at T = -36°C. The reaction was quenched by evaporation and subjected to NMR and GPC analysis.

### Example 11:

First the diborane **(*R*)-II-7** (0.034 mmol, 4 eq.) was dissolved in PO (0.987 g, 17.04 mmol, 2000 eq.). Subsequently, the initiator (BnOH, 0.0425 mmol, 5 eq.) and the corresponding organobase **(III-11,** 0.0085 mmol, 1 eq.) were added to result in a total molar ratio of BA/LA/IN/PO = 1:4:5:2000. The reaction was stirred for 2 h at T = 25°C. The reaction was quenched by evaporation and subjected to NMR and GPC analysis.

### Example 12:

First the diborane **(*R*)-II-7** (0.017 mmol, 2 eq.) was dissolved in PO (0.495 g, 8.52 mmol, 1000 eq.) and THF (42.7 mL). Subsequently, the initiator (BnOH, 0.0213 mmol, 2.5 eq.) and the corresponding organobase **(III-19,** 0.0085 mmol, 1 eq.) were added to result in a total molar ratio of BA/LA/IN/PO = 1:2:2.5:1000 and an initial monomer concentration of 2mol/L. The reaction was stirred for 8 d at T = 25°C. The reaction was quenched by evaporation and subjected to NMR and GPC analysis.

### Example 13:

First the diborane **(*R*)-II-7** (0.017 mmol, 2 eq.) was dissolved in BO (0.740 g, 8.52 mmol, 1000 eq.). Subsequently, the initiator (BnOH, 0.0213 mmol, 2.5 eq.) and the corresponding organobase **(III-19,** 0.0085 mmol, 1 eq.) were added to result in a total molar ratio of BA/LA/IN/PO = 1:2:2.5:1000. The reaction was stirred for 24 h at T = 25°C. The reaction was quenched by evaporation and subjected to NMR and GPC analysis.

### Example 14:

First the diborane **(*R*)-II-7** (0.017 mmol, 2 eq.) was dissolved in AGE (1.01 g, 8.52 mmol, 1000 eq.). Subsequently, the initiator (BnOH, 0.0213 mmol, 2.5 eq.) and the corresponding organobase **(III-19,** 0.0085 mmol, 1 eq.) were added to result in a total molar ratio of BA/LA/IN/PO = 1:2:2.5:1000. The reaction was stirred for 24 h at T = 25°C. The reaction was quenched by evaporation and subjected to NMR and GPC analysis.

## Claims

1. Catalyst system for the synthesis of it-polyethers from epoxide monomers, comprising an organobase BA, a diborane Lewis acid LA and optionally an initiator IN, wherein it-polyethers denote isotactic enriched polyethers, wherein the proportion of isotactic diad placement (m) is > 50%.

2. Catalyst system according to claim 1, wherein the diborane Lewis acid LA is a Lewis acid having the general formula I 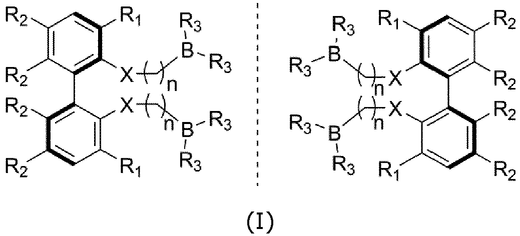
wherein R₁ mutually independently represents H, C₁-C₁₀ alkyl, C₂-C₁₀ alkenyl, C₃-C₁₂ cycloalkyl, C₆-C₁₀₀ polyoxyalkylene, C₅-C₁₀ aryl or C₅-C₁₀ heteroaryl, wherein the C₅-C₁₀ aryl or C₅-C₁₀ heteroaryl may be further substituted;
R₂ and R₃ mutually independently represent H, C₁-C₁₀ alkyl, C₂-C₁₀ alkenyl, C₃-C₁₂ cycloalkyl, C₆-C₁₀₀ polyoxyalkylene, C₅-C₁₀ aryl or C₅-C₁₀ heteroaryl, wherein the C₅-C₁₀ aryl or C₅-C₁₀ heteroaryl may be further substituted;
wherein if the C₅-C₁₀ aryl or C₅-C₁₀ heteroaryl mentioned above is further substituted, the further substituent is preferably an alkyl- and more preferably a C₁₋₆-alkyl substituent, wherein preferably the substituted C₅-C₁₀ aryls or C₅-C₁₀ heteroaryls have 1 to 3 substituents;
wherein X is -O-, -S-, -CH₂-, -NR₅- or -PR₅, and R₅ has the meaning given above for R₁;
wherein n determines the number of methylene (-CH₂-) repeat units, wherein preferably n is an integer of from 1 to 10;
and wherein the diborane Lewis acid is enantiomerically pure or a racemic mixture.

3. Catalyst system according to claim 2, R₁ mutually independently represents H, methyl, ethyl, n-propyl, isopropyl, tert-butyl, neopentyl, isoamyl, cyclohexyl, phenyl, 2,6-dimethylphenyl, 2,6-diisopropylphenyl or mesityl moieties;
R₂ mutually independently represents H, methyl, ethyl, n-propyl, isopropyl, tert-butyl or the two R₂ together with the phenyl ring, to which they are attached, form a naphthyl group, wherein preferably all R₂ in the formula (I) form naphthyl groups;
R₃ mutually independently represents H, methyl, ethyl, n-propyl, isopropyl, tert-butyl or, together with the B-atom to which they are attached form a borabicyclo[3.3.1]nonane group;
X mutually independently represents O or CH₂;
and n mutually independently represents 1 or 3 methylene (-CH₂-) units.

4. Catalyst system according to claim 2 or 3, wherein the diborane Lewis acid (LA) is one or more compounds having the formulae (II-1), (II-2), (II-3), (II-4), (II-5), (II-6), (II-7) and (II-8) shown below, whereby these structures are either enantiomerically pure or racemic compounds.

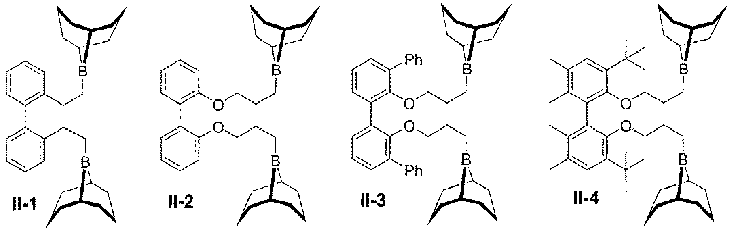

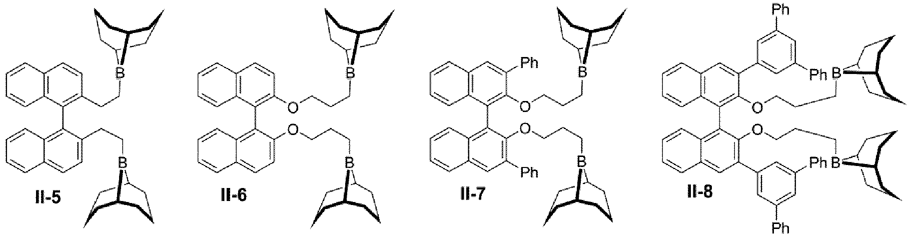

5. Catalyst system according to one of claims 1 to 4, wherein the organobase (BA) is one or more of the compounds (III-1), (III-2), (III-3), (III-4), (III-5), (III-6), (III-7), (III-8), (III-9), (III-10), (III-11), (III-12), (III-13), (III-14), (III-15), (III-16), (III-17), (III-18), (III-19), (III-20), (III-21) and (III-22).

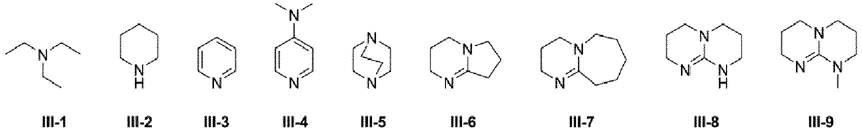

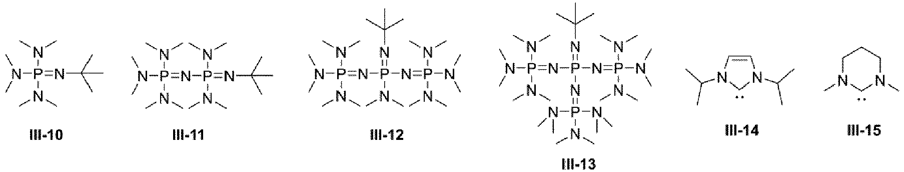

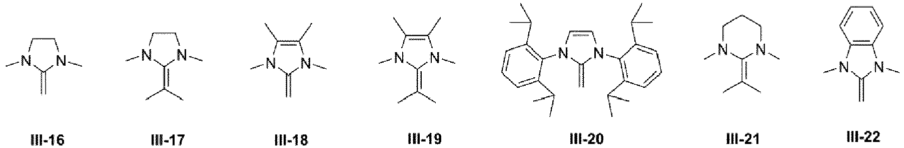

6. Catalyst system according to one of claims 1 to 5, wherein the initiator IN is a compound of the general structure R-YH, whereby Y is -O-, -S-, -NH- or COO and R is selected from polymeric residues, preferably polyether-, polyester- or polyamide residues, C₁-C₁₀ alkyl, C₂-C₁₀ alkenyl, C₃-C₁₂ cycloalkyl, C₆-C₁₀₀ polyoxyalkylene, C₅-C₁₀ aryl, benzyl or C₅-C₁₀ heteroaryl, wherein the C₅-C₁₀ aryl or C₅-C₁₀ heteroaryl may be further substituted, wherein preferably the initiator IN is selected from one or more of the group consisting of benzyl alcohol, butan(di)ol, methoxyPEG, HO-PEG-OH (PEG = polyethylene glycol, preferably with an Mn of 100-35.000 g/mol) or polycaprolactone-OH.

7. Catalyst system according to one of claims 1 to 6, wherein the molar ratio of BA to LA is in the range from 1 : 0.5 to 1 : 50, more preferably in the range from 1 : 0.5 to 1 : 10, and most preferably in the range from 1 : 1 to 1 : 4; and wherein the molar ratio of IN to either BA or LA is 50:1 to 1:1, in a more preferably 25:1 to 1:1, and most preferably 5:1 to 1:1.

8. Process for the production of *isotactic* and isotactic-enriched (*it-*)polyethers, wherein the process comprises a step of reacting an epoxide monomer in the presence of the catalyst according to one of claims 1 to 7.

9. Process for the production of a block copolymer containing at least one block of an it-polyether, wherein the process comprises a step of reacting an epoxide monomer and a polymeric initiator are in the presence of the catalyst according to one of claims 1 to 7.

10. Process according to claim 8 or 9, wherein the reaction temperature is in the range of from -50°C to 100°C, preferably in the range from -35°C to 60°C, more preferably in the range from 0°C to 25°C.

11. Process according to one of claims 8 or 9, wherein the epoxide compound is one or more compounds selected from the group consisting of 4-tert-butylphenyl glycidyl ether, phenyl glycidyl ether, 1-naphthyl glycidyl ether, 2-naphthyl glycidyl ether, 4-chlorophenyl glycidyl ether, 2,4,6-trichlorophenyl glycidyl ether, 2,4,6-tribromophenyl glycidyl ether, pentafluorophenyl glycidyl ether, cyclohexyl glycidyl ether, benzyl glycidyl ether, glycidyl benzoate, glycidyl acetate, glycidyl cyclohexylcarboxylate, methyl glycidyl ether, ethyl glycidyl ether, butyl glycidyl ether, hexyl glycidyl ether, 2-ethylhexyl glycidyl ether, octyl glycidyl ether, C10 - C18 alkyl glycidyl ether, allyl glycidyl ether, ethylene oxide, propylene oxide, styrene oxide, 1,2-butene oxide, 2,3-butene oxide, 1,2-hexene oxide, oxides of C10 - C18 alpha-olefins, cyclohexene oxide, vinylcyclohexene monoxide, limonene monoxide, and/or butadiene monoepoxide, preferably, wherein the epoxide is one or more compounds selected from the group consisting of ethylene oxide, propylene oxide, butylene oxide, hexylene oxide, allyl glycidyl ether, 2-ethylhexyl glycidyl ether, and most preferably, wherein the epoxide is selected from propylene oxide and/or butylene oxide.

12. Polyether, obtainable or obtained by the process of any one of claims 8 to 11, wherein the degree of isotacticity (isotactic diad placement, %m) of the polyether is preferably ≥ 50%, more preferably ≥ 60% and even more preferably ≥ 70%, as determined by ¹³C nuclear magnetic resonance (NMR) analysis.

13. Use of a catalyst system according to any one of claims 1 to 7 for the production of it-polyethers.

## Patentansprüche

1. Katalysatorsystem für die Synthese von *it*-Polyethern aus Epoxidmonomeren, umfassend eine Organobase BA, eine Diboran-Lewis-Säure LA und gegebenenfalls einen Starter IN, wobei *it*-Polyether isotaktisch angereicherte Polyether bezeichnen, wobei der Anteil der isotaktischen Diadenplatzierung (*m*) > 50 % ist.

2. Katalysatorsystem nach Anspruch 1, wobei die Diboran-Lewis-Säure LA eine Lewis-Säure mit der allgemeinen Formel I ist 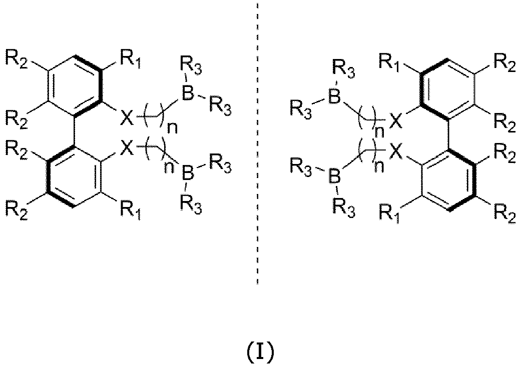
wobei R₁ unabhängig voneinander H, C₁-C₁₀-Alkyl, C₂-C₁₀-Alkenyl, C₃-C₁₂-Cycloalkyl, C₆-C₁₀₀-Polyoxyalkylen, C₅-C₁₀-Aryl oder C₅-C₁₀-Heteroaryl darstellt, wobei das C₅-C₁₀-Aryl oder C₅-C₁₀-Heteroaryl weiter substituiert sein kann;
R₂ und R₃ unabhängig voneinander H, C₁-C₁₀-Alkyl, C₂-C₁₀-Alkenyl, C₃-C₁₂-Cycloalkyl, C₆-C₁₀₀-Polyoxyalkylen, C₅-C₁₀-Aryl oder C₅-C₁₀-Heteroaryl darstellen, wobei das C₅-C₁₀-Aryl oder C₅-C₁₀-Heteroaryl weiter substituiert sein kann;
wobei, wenn das oben erwähnte C₅-C₁₀-Aryl oder C₅-C₁₀-Heteroaryl weiter substituiert ist, der weitere Substituent vorzugsweise ein Alkyl- und mehr bevorzugt ein C₁₋₆-Alkylsubstituent ist, wobei die substituierten C₅-C₁₀-Aryle oder C₅-C₁₀-Heteroaryle 1 bis 3 Substituenten aufweisen;
wobei X -O-, -S-, -CH₂-, -NR₅- oder -PR₅ ist, und R₅ die vorstehend für R₁ angegebene Bedeutung hat;
wobei n die Anzahl der Methylen-Wiederholungseinheiten (-CH₂-) bestimmt, wobei n vorzugsweise eine ganze Zahl von 1 bis 10 ist;
und wobei die Diboran-Lewis-Säure enantiomerenrein oder ein racemisches Gemisch ist.

3. Katalysatorsystem nach Anspruch 2, R₁ stellt unabhängig voneinander H, Methyl-, Ethyl-, n-Propyl-, Isopropyl-, tert-Butyl-, Neopentyl-, Isoamyl-, Cyclohexyl-, Phenyl-, 2,6-Dimethylphenyl-, 2,6-Diisopropylphenyl- oder Mesitylreste dar;
R₂ stellt unabhängig voneinander H, Methyl, Ethyl, n-Propyl, Isopropyl, tert-Butyl dar oder die beiden R₂ bilden zusammen mit dem Phenylring, an den sie gebunden sind, eine Naphthylgruppe, wobei vorzugsweise alle R₂ in der Formel (I) Naphthylgruppen bilden;
R₃ stellt unabhängig voneinander H, Methyl, Ethyl, n-Propyl, Isopropyl, tert-Butyl dar oder bilden zusammen mit dem B-Atom, an das sie gebunden sind, eine Borabicyclo[3.3.1]nonan-Gruppe;
X stellt unabhängig voneinander O oder CH₂ dar;
und n stellt unabhängig voneinander 1 oder 3 Methyleneinheiten (-CH₂-) dar.

4. Katalysatorsystem nach Anspruch 2 oder 3, wobei die Diboran-Lewis-Säure (LA) eine oder mehrere Verbindungen mit den nachstehend gezeigten Formeln (II-1), (II-2), (II-3), (II-4), (II-5), (II-6), (II-7) und (II-8) ist, wobei diese Strukturen entweder enantiomerenreine oder racemische Verbindungen sind.

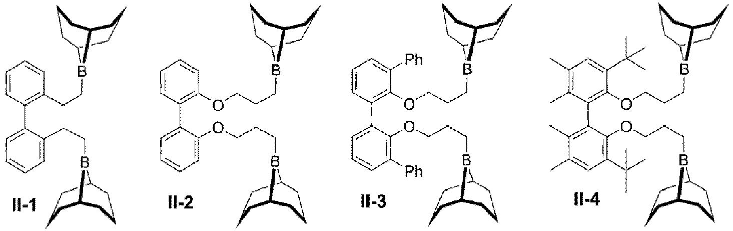

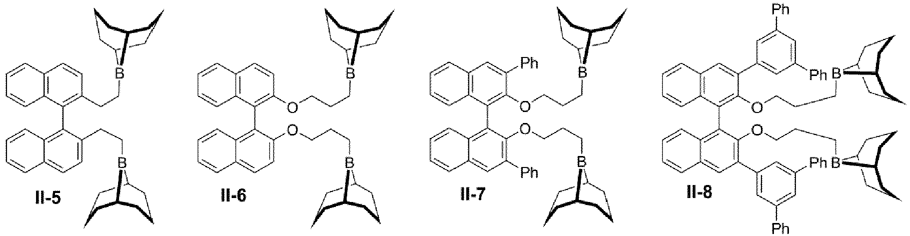

5. Katalysatorsystem nach einem der Ansprüche 1 bis 4, wobei die Organobase (BA) eine oder mehrere der Verbindungen (III-1), (III-2), (III-3), (III-4), (III-5), (III-6), (III-7), (III-8), (III-9), (III-10), (III-11), (III-12), (III-13), (III-14), (III-15), (III-16), (III-17), (III-18), (III-19), (III-20), (III-21) und (III-22) ist.

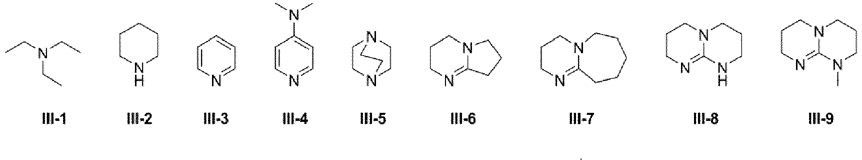

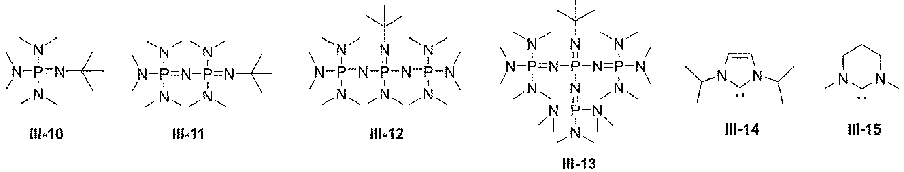

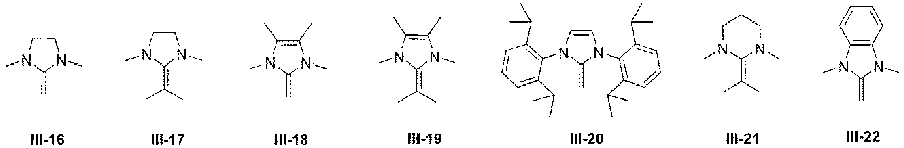

6. Katalysatorsystem nach einem der Ansprüche 1 bis 5, wobei der Starter IN eine Verbindung der allgemeinen Struktur R-YH ist, wobei Y -O-, -S-, -NH- oder COO ist und R aus polymeren Resten, vorzugsweise Polyether-, Polyester- oder Polyamidresten, C₁-C₁₀-Alkyl, C₂-C₁₀-Alkenyl, C₃-C₁₂-Cycloalkyl, C₆-C₁₀₀-Polyoxyalkylen, C₅-C₁₀-Aryl, Benzyl oder C₅-C₁₀-Heteroaryl ausgewählt ist, wobei das C₅-C₁₀-Aryl oder C₅-C₁₀-Heteroaryl weiter substituiert sein kann, wobei der Starter IN vorzugsweise aus einem oder mehreren der Gruppe bestehend aus Benzylalkohol, Butan(di)ol, MethoxyPEG, HO-PEG-OH (PEG = Polyethylenglykol, vorzugsweise mit einem Mn von 100-35.000 g/mol) oder Polycaprolacton-OH ausgewählt ist.

7. Katalysatorsystem nach einem der Ansprüche 1 bis 6, wobei das Molverhältnis von BA zu LA im Bereich von 1:0,5 bis 1:50, mehr bevorzugt im Bereich von 1:0,5 bis 1:10 und am meisten bevorzugt im Bereich von 1:1 bis 1:4 liegt; und wobei das Molverhältnis von IN zu entweder BA oder LA 50:1 bis 1:1, mehr bevorzugt 25:1 bis 1:1 und am meisten bevorzugt 5:1 bis 1:1 beträgt.

8. Verfahren zur Herstellung von *isotaktischen* und isotaktisch-anreicherten (it-)Polyethern, wobei das Verfahren einen Schritt umfasst, bei dem ein Epoxidmonomer in Gegenwart des Katalysators nach einem der Ansprüche 1 bis 7 zur Reaktion gebracht wird.

9. Verfahren zur Herstellung eines Blockcopolymers, enthaltend mindestens einen Block eines *it*-Polyethers, wobei das Verfahren einen Schritt des Umsetzens eines Epoxidmonomers und eines polymeren Starters in Gegenwart des Katalysators nach einem der Ansprüche 1 bis 7 umfasst.

10. Verfahren nach Anspruch 8 oder 9, wobei die Reaktionstemperatur im Bereich von -50 °C bis 100 °C liegt, vorzugsweise im Bereich von -35 °C bis 60 °C, mehr bevorzugt im Bereich von 0 °C bis 25 °C.

11. Verfahren nach einem der Ansprüche 8 oder 9, wobei die Epoxidverbindung eine oder mehrere Verbindungen ist, die ausgewählt sind aus der Gruppe bestehend, 4-tert-Butylphenylglycidylether, Phenylglycidylether, 1-Naphthylglycidylether, 2-Naphthylglycidylether, 4-Chlorphenylglycidylether, 2,4,6-Trichlorphenylglycidylether, 2,4,6-Tribromphenylglycidylether, Pentafluorphenylglycidylether, Cyclohexylglycidylether, Benzylglycidylether, Glycidylbenzoat, Glycidylacetat, Glycidylcyclohexylcarboxylat, Methylglycidylether, Ethylglycidylether, Butylglycidylether, Hexylglycidylether, 2-Ethylhexylglycidylether, Octylglycidylether, C10-C18-Alkylglycidylether, Allylglycidylether, Ethylenoxid, Propylenoxid, Styroloxid, 1,2-Butenoxid, 2,3-Butenoxid, 1,2-Hexenoxid, Oxide von C10-C18-alpha-Olefinen, Cyclohexenoxid, Vinylcyclohexenmonoxid, Limonenmonoxid und/oder Butadienmonoepoxid, vorzugsweise wobei das Epoxid eine oder mehrere Verbindungen ist, die ausgewählt sind aus der Gruppe bestehend aus Ethylenoxid, Propylenoxid, Butylenoxid, Hexylenoxid, Allylglycidylether, 2-Ethylhexylglycidylether, und am meisten bevorzugt wobei das Epoxid ausgewählt ist aus Propylenoxid und/oder Butylenoxid.

12. Polyether, erhaltbar oder erhalten durch das Verfahren nach einem der Ansprüche 8 bis 11, wobei der Grad der Isotaktizität (isotaktische Diadenplatzierung, %m) des Polyethers vorzugsweise ≥ 50 %, mehr bevorzugt ≥ 60 % und noch mehr bevorzugt ≥ 70 % beträgt, bestimmt mittels ¹³C-Kernspinresonanzanalyse (NMR-Analyse).

13. Verwendung eines Katalysatorsystems nach einem der Ansprüche 1 bis 7 zur Herstellung von *it*-Polyethern.

## Revendications

1. Système catalytique pour la synthèse de polyéthers it à partir de monomères d'époxyde, comprenant une base organique BA, un acide de Lewis LA de type diborane et facultativement un initiateur IN, dans lequel « polyéthers it » signifie « polyéthers à enrichissement isotactique », dans lequel la proportion de placement de diade isotactique (m) est > 50 %.

2. Système catalytique selon la revendication 1, dans lequel l'acide de Lewis LA de type diborane est un acide de Lewis ayant la formule générale 1 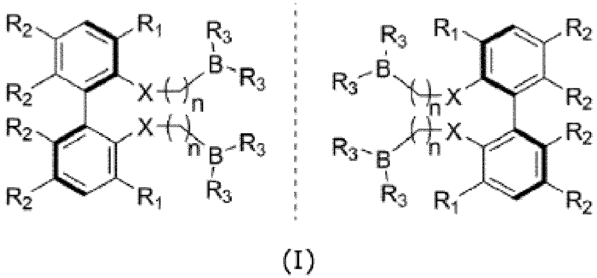
dans lequel les R₁ indépendamment l'un de l'autre représentent H, un alkyle en C₁-C₁₀, un alcényle en C₂-C₁₀, un cycloalkyle en C₃-C₁₂, un polyoxyalkylène en C₆-C₁₀₀, un aryle en C₅-C₁₀ ou un hétéroaryle en C₅-C₁₀, dans lequel l'aryle en C₅-C₁₀ ou l'hétéroaryle en C₅-C₁₀ peut être en outre substitué ;
R₂ et R₃ indépendamment l'un de l'autre représentent H, un alkyle en C₁-C₁₀, un alcényle en C₂-C₁₀, un cycloalkyle en C₃-C₁₂, un polyoxyalkylène en C₆-C₁₀₀, un aryle en C₅-C₁₀ ou un hétéroaryle en C₅-C₁₀, dans lequel l'aryle en C₅-C₁₀ ou l'hétéroaryle en C₅-C₁₀ peut être en outre substitué ;
dans lequel si l'aryle en C₅-C₁₀ ou l'hétéroaryle en C₅-C₁₀ mentionné ci-dessus est en outre substitué, le substituant supplémentaire est de préférence un substituant alkyle et de manière davantage préférée un substituant alkyle en C₁-Ce, dans lequel de préférence les aryles en C₅-C₁₀ ou hétéroaryles en C₅-C₁₀ substitués ont 1 à 3 substituants ; dans lequel X est -O-, -S-, -CH₂-, -NR₅- ou -PR₅, et R₅ a la signification donnée ci-dessus pour R₁ ;
dans lequel n détermine le nombre de motifs répétitifs méthylène (-CH₂-), dans lequel de préférence n est un nombre entier de 1 à 10 ;
et dans lequel l'acide de Lewis de type diborane est énantiomériquement pur ou un mélange racémique.

3. Système catalytique selon la revendication 2, les R₁ indépendamment l'un de l'autre représentent H, un méthyle, un éthyle, un n-propyle, un isopropyle, un tert-butyle, un néopentyle, un isoamyle, un cyclohexyle, un phényle, un 2,6-diméthylphényle, un 2,6-diisopropylphényle ou un mésityle ;
les R2 indépendamment les uns des autres représentent H, un méthyle, un éthyle, un n-propyle, un isopropyle, un tert-butyle ou les deux R₂ conjointement avec le cycle phényle auquel ils sont liés forment un groupe naphtyle, dans lequel de préférence tous les R₂ dans la formule (I) forment des groupes naphtyle ;
les R₃ indépendamment les uns des autres représentent H, un méthyle, un éthyle, un n-propyle, un isopropyle, un tert-butyle ou, conjointement avec l'atome B auquel ils sont liés forment un groupe borabicyclo[3.3.1]nonane ;
les X indépendamment l'un de l'autre représentent O ou CH₂ ;
et les n indépendamment l'un de l'autre représentent 1 ou 3 motifs méthylène (-CH₂-).

4. Système catalytique selon la revendication 2 ou 3, dans lequel l'acide de Lewis (LA) de type diborane est un ou plusieurs composés ayant les formules (11-1), (II-2), (II-3), (II-4), (II-5), (II-6), (II-7) et (II-8) présentées ci-dessous, moyennant quoi ces structures sont soit des composés énantiomériquement purs, soit des composés racémiques.

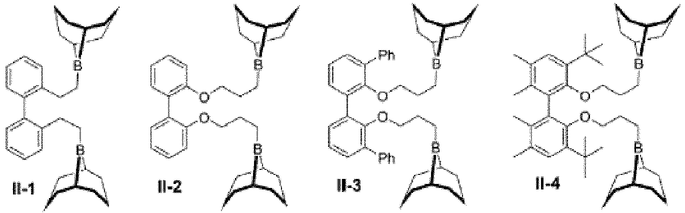

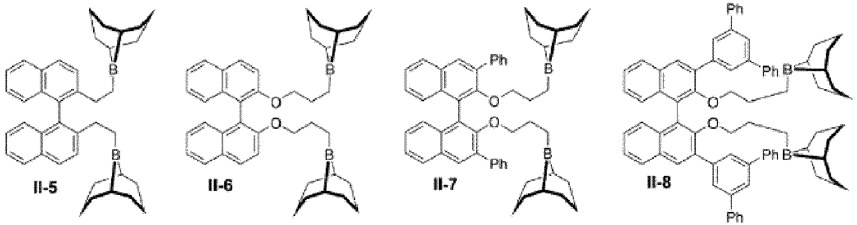

5. Système catalytique selon l'une des revendications 1 à 4, dans lequel la base organique (BA) est une ou plusieurs parmi les composés (III-1), (III-2), (III-3), (III-4), (III-5), (III-6), (III-7), (III-8), (III-9), (III-10), (III-11), (III-12), (III-13), (III-14), (III-15), (III-16), (III-17), (III-18), (III-19), (III-20), (III-21) et (III-22).

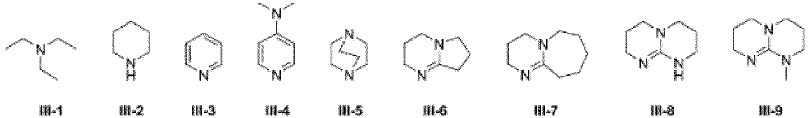

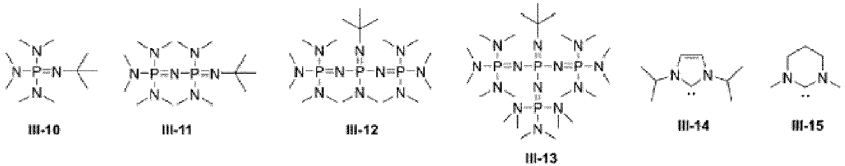

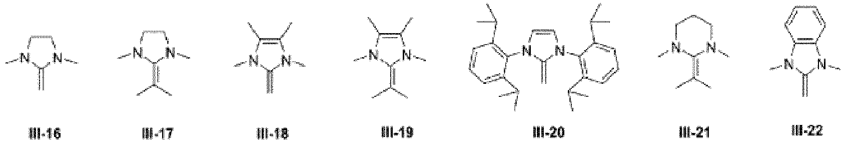

6. Système catalytique selon l'une des revendications 1 à 5, dans lequel l'initiateur IN est un composé de structure générale R-YH, moyennant quoi Y représente -O-, -S-, -NH- ou COO et R est sélectionné parmi des résidus polymères, de préférence des résidus polyéther, polyester ou polyamide, un alkyle en C₁-C₁₀, un alcényle en C₂-C₁₀, un cycloalkyle en C₃-C₁₂, un polyoxyalkylène en C₆-C₁₀₀, un aryle en C₅-C₁₀, un benzyle ou un hétéroaryle en C₅-C₁₀, dans lequel l'aryle en C₅-C₁₀ ou l'hétéroaryle en C₅-C₁₀ peut être en outre substitué, dans lequel de préférence l'initiateur IN est sélectionné parmi un ou plusieurs du groupe consistant en l'alcool benzylique, le butan(di)ol, un méthoxyPEG, HO-PEG-OH (PEG = polyéthylène glycol, de préférence avec une Mn de 100 à 35 000 g/mol) ou une polycaprolactone-OH.

7. Système catalytique selon l'une des revendications 1 à 6, dans lequel le rapport molaire BA sur LA est dans la plage de 1:0,5 à 1:50, de manière davantage préférée dans la plage de 1:0,5 à 1:10, et de manière préférée entre toutes dans la plage de 1:1 à 1:4 ; et dans lequel le rapport molaire IN sur BA ou LA est de 50:1 à 1:1, de manière davantage préférée de 25:1 à 1:1, et de manière préférée entre toutes de 5:1 à 1:1.

8. Procédé de production de polyéthers *isotactiques* et à *enrichissement isotactique* (it), dans lequel le procédé comprend une étape de réaction d'un monomère d'époxyde en présence du catalyseur selon l'une des revendications 1 à 7.

9. Procédé de production d'un copolymère séquencé contenant au moins une séquence d'un polyéther it, dans lequel le procédé comprend une étape de réaction d'un monomère d'époxyde et d'un initiateur polymère en présence du catalyseur selon l'une des revendications 1 à 7.

10. Procédé selon la revendication 8 ou 9, dans lequel la température réactionnelle est dans la plage de -50 °C à 100 °C, de préférence dans la plage de -35 °C à 60 °C, de manière davantage préférée dans la plage de 0 à 25 °C.

11. Procédé selon l'une des revendications 8 ou 9, dans lequel le composé d'époxyde est un ou plusieurs composés sélectionnés dans le groupe consistant en le 4-tert-butylphénylglycidyléther, le phénylglycidyléther, le 1-naphtylglycidyléther, le 2-naphtylglycidyléther, le 4-chlorophénylglycidyléther, le 2,4,6-trichlorophényl-glycidyléther, le 2,4,6-tribromophénylglycidyléther, le pentafluorophénylglycidyléther, le cyclohexylglycidyl-éther, le benzylglycidyléther, le benzoate de glycidyle, l'acétate de glycidyle, le cyclohexylcarboxylate de glycidyle, le méthylglycidyléther, l'éthylglycidyléther, le butylglycidyléther, l'hexylglycidyléther, le 2-éthyl-hexylglycidyléther, l'octylglycidyléther, un (alkyle en C₁₀-C₁₈) glycidyléther, l'allylglycidyléther, l'oxyde d'éthylène, l'oxyde de propylène, l'oxyde de styrène, l'oxyde de 1,2-butène, l'oxyde de 2,3-butène, l'oxyde de 1,2-hexène, les oxydes d'alpha-oléfines en C₁₀ à C₁₈, l'oxyde de cyclohexène, le monoxyde de vinylcyclohexène, le monoxyde de limonène, et/ou le monoépoxyde de butadiène, de préférence, dans lequel l'époxyde est un ou plusieurs composés sélectionnés dans le groupe consistant en l'oxyde d'éthylène, l'oxyde de propylène, l'oxyde de butylène, l'oxyde d'hexylène, l'allylglycidyléther, le 2-éthylhexyl-glycidyléther, et de manière préférée entre toutes, dans lequel l'époxyde est sélectionné parmi l'oxyde de propylène et/ou l'oxyde de butylène.

12. Polyéther, pouvant être obtenu ou obtenu par le procédé selon l'une quelconque des revendications 8 à 11, dans lequel le degré d'isotacticité (placement de diade isotactique, %m) du polyéther est de préférence ≥ 50 %, de manière davantage préférée ≥ 60 % et de manière encore plus préférée ≥ 70 %, tel que déterminé par une analyse par résonance magnétique nucléaire (RMN) ¹³C.

13. Utilisation d'un système catalytique selon l'une quelconque des revendications 1 à 7 pour la production de polyéthers it.
